# EUROPEAN PATENT SPECIFICATION

(11) **EP 0 812 220 B1**
(45) Date of publication and mention of the grant of the patent: **19.05.2004**
(21) Application number: 95916117.5
(22) Date of filing: 29.03.1995
(51) Int. Cl.: A61M 16/00, A62B 9/06, A61M 16/04

(54) **ACCESS CONTROL RESPIRATORY DEVICES**
BEATMUNGSVORRICHTUNGEN MIT KONTROLLIERTEM ZUGANG
DISPOSITIFS D'ACCES CONTROLE AU SYSTEME RESPIRATOIRE

(30) Priority: 28.02.1995 US 395351
(43) Date of publication of application: 17.12.1997
(73) Proprietor: BALLARD MEDICAL PRODUCTS, Draper, UT 84020 (US)
(72) Inventor: LORENZEN, Rick, D., Ogden, UT 84401 (US); MADSEN, Edward, B., Riverton, UT 84065 (US)
(74) Representative: Davies, Christopher Robert
(86) International application number: PCT/US1995/003756
(87) International publication number: WO 1996/026757

(56) References cited:
- DE-A- 2 920 366
- DE-A- 2 939 794
- US-A- 3 423 063
- US-A- 5 025 806
- US-A- 5 029 580
- US-A- 5 065 754
- US-A- 5 158 569
- US-A- 5 259 376
- US-A- 5 354 267

## Description

The invention disclosed herein relates generally to improved medical care for intubated patients, and more particularly to a novel multiple access manifold, fitting, or an adapter and access control device for ventilating, aspirating, monitoring, sampling, and providing therapeutic delivery to the respiratory tracts of intubated medical patients, including infants, adolescents, and adults.

### Background Art

Respiratory patient care is a dynamically developing field in medicine, ranging in its needs from infants to the aged. The range of respiratory ailments, both temporary and permanent, to which such patients are subjected, are many and varied. The frontier of medical knowledge is advancing and recommended treatments have become a blend of old and more recent discoveries.

Most problems now center or focus on multiple needs of the patient and accommodation of multiple treatments, some to be performed at the same time. The lack of equipment to facilely, efficiently, and safely accomplish the multiple therapies in the best interest of the patient has been and continues to be a concern. Other equipment problems also exist which concern preventing cost-oriented, unsafe extended use of ventilating, aspirating, and other respiratory access apparatus, reliability during use, quick and reliable removal and exchange of spent aspirating and ventilating devices without compromising the quality of health care provided to the patient, avoiding intentional or inadvertent conversion from a closed system to an open system, prevention of stress and/or occlusion of flow passageways to and from the patient's respiratory system, avoidance of a large inventory of a variety of incompatible products, providing easy, fail-safe access for multiple purposes.

By way of an example only, with low lung capacity patients, such as premature babies and adults suffering from emphysema, one problem is the removal of accumulated lung secretions without starving the patient for oxygen (thereby causing undesirable side effects) during the secretion removal process.

Sight must not be lost as to the deficiencies in prior proposals in terms of risks created for the health care provider. Largely, proposals of the past have ignored the needs of the health care provider to receive a reasonable measure of protection from contamination by the patient.

Providing apparatus and methodology having the capacity to promptly, efficiently, safely, and cost effectively address the health care needs of intubated patients across the entire spectrum of respiratory ailments comprises, prior to the present invention, a largely unresolved need. The range of procedures comprise: ventilating, aspiration, oxygenation, sampling, visual inspection, in-line sensing, pressure monitoring, flushing, and medication and/or lavage. Better protection for the health care provider has been a long-term unsatisfied need.

A relevant prior art device in this respect is disclosed in DE-A-2 939 794.

### Disclosure of the Invention

In brief summary, the present invention substantially alleviates problems of the prior art and comprises apparatus by which a closed ventilating system accommodates multiple access to the respiratory system of an intubated medical patient without compromising the closed character of the system and without interruption of the flow of ventilating gases to the patient. Access to the respiratory system through one or more access sites of the closed system apparatus is provided to ventilate the lungs of the patient with gas or gases, to aspirate secretions from the lungs, to oxygenate the lungs to eliminate or reduce residual CO₂ therefrom, to visually inspect selected parts of the respiratory system, to sample sputum and gases, to sense parameters such as flow rates, pressure, and temperature, to flush with washing solution, and/or to administer medication, gases, and/or lavage.

One system is unitized into severable and disposable components which are cost effective and accommodate good health care practices while promoting limitations on duration of use well within appropriate medical tolerances. Quick removal and replacement of discarded components is accommodated without introduction of additional risks to the patient. The technology of the present invention has substantial universal application to all respiratory patients, ranging from infants to the aged. An accessory device can be added or removed from a manifold, fitting, or adapter while the associated access port is in an off condition. Novel access control devices in association with an access port of a manifold, fitting, or adapter accommodate selective enabled and disabled of port of a manifold, fitting, or adapter for access and non-access and for attachment, detachment, and exchange of accessory devices.

With the foregoing in mind, it is a primary object of the present invention to substantially alleviate problems of the prior art in the field of respiratory care for medical patients.

According to the present invention, there is thus provided an adaptor assembly as claimed in claim 1.

It is an additional dominant object of the present invention to provide apparatus by which a closed ventilating system is able to accommodate multiple access to the respiratory system of an intubated medical patient.

An additional paramount object is the provision of novel apparatus by which a closed ventilating system accommodates multiple access to the respiratory system of an intubated medical patient without compromising the closed character of the system.

An additional object of the present invention is the provision of access through an access site in a closed system respiratory apparatus to accommodate ventilating of the lungs of the patient with gas or gases, to aspirate secretions from the lungs, to oxygenate the lungs to eliminate or reduce residual carbon dioxide therefrom, to visually inspect selected parts of the respiratory system, to sample sputum and gases, to sense parameters such as flow rates, pressure, and temperature, to flush with washing solution and/or to administer medication, gases, and/or lavage, and related methods.

An additional significant object is the provision of a closed respiratory health care system unitized into severable and disposable components which are cost effective and accommodate good health care practices while promoting limitations on duration of use well within appropriate medical tolerances.

It is an additional valuable object to provide for quick removal and replacement of discardable components toward the end of their useful life in a respiratory health care system and to accommodate such without introduction of additional risks to the patient.

It is another dominant object to provide a respiratory health care system which has substantial universal application to all respiratory patients ranging from infants to the aged.

It is a further object of significance to provide a respiratory health care system by which superior protection is provided by the care giver to an intubated patient.

It is a further object of the present invention to provide novel respiratory systems
comprising a plurality of entry sites normally sealed to minimize introduction of contamination and avoid loss of ventilating gas or pressure, but yet to allow selective entry of an instrument, device, or the like.

It is a primary object of the present invention to provide a unitized respiratory health care system
where components of the system comprise disposable segments which may be facilely connected and removed by the care giver.

A further important object of the present invention is to provide features in a respiratory health care system which avoid imposition of stress on the components and prohibit occlusion of flow pathways.

It is a prominent object of the present invention to provide respiratory health care systems
which accommodate simultaneous access to and treatment within the respiratory system of a medical patient.

It is a further object of the present invention to provide novel respiratory health care systems having minimal dead space.

Still another paramount object is the provision of a mechanism in which access and non-access of one or more accessory devices to the interior of a manifold, fitting, or adapter is selectively enabled and disabled.

Still a further valuable object is the provision of novel access port control devices
by which accessory devices are selectively placed in and out of communication with the access port of a manifold, fitting, or adapter.

Another object of significance is the provision of novel manifold access port control devices
by which a connector site of the control device can be disabled to accommodate attachment, detachment, non-use, or exchange of accessory devices and which can be enabled for use of an attached ancillary device in respect to the access port of the manifold, fitting, or adapter.

A further object is the provision of novel access control appliances in the nature of unique plugs for access ports which accommodate various accessory devices, the distal ends of which may comprise differing diameters.

These and other objects and features of the present invention will be apparent from the detailed description taken with reference to the accompanying drawings.

### Description of the Drawings

Figure 1 is a perspective, with some parts shown in cross-section for clarity, of one multi-access manifold, fitting, or adapter comprising an access control device in the form of a turret valve with an aspirating catheter assembly and suction valve attached hereto;
Figure 2 is a fragmentary exploded perspective of the apparatus illustrated in Figure 1;
Figure 3A is a cross-sectional view taken along lines 3A-3A of Figure 1;
Figure 3B is a cross-section similar to Figure 3A, but showing a somewhat modified rotor of an access control device;
Figure 4 is a side elevation view of another multi-access turret valve configuration embodying principles of the present invention;
Figure 5 is a fragmentary cross-section of the turret valve of Figure 4 with the cap shown in its inserted position preparatory to receiving an accessory device;
Figure 6 is a fragmentary perspective representation of the turret valve of Figure 4 and 5;
Figure 7 is a fragmentary cross-section of still another turret valve configuration, also embodying principles of the present invention;
Figure 8 is a frontal elevation of still another turret valve configuration;
Figure 9 is a perspective of another access control device in the form of a slider valve, embodying principles of the present invention;
Figure 10 is an enlarged exploded cross-section of the slider valve of Figure 9 taken along lines 10-10 of Figure 9;
Figure 11 is an enlarged exploded perspective of the slider valve of Figure 9;
Figure 12A is a cross-section of still another configuration of a manifold, fitting, or adapter equipped with a turret access control device;
Figure 12B is an enlarged fragmentary cross-section of the turret access control device of Figure 12A;
Figures 13, 14A and 14B are perspectives of the rotor and the proximal and distal faces of the stator of a further turret valve embodying principles of the present invention; and
Figure 15 is a fragmentary view of an access control appliance in the nature of a plug for an access port which accommodates various accessory devices the distal ends of which may comprise differing diameters.

### Modes for Carrying Out the Invention

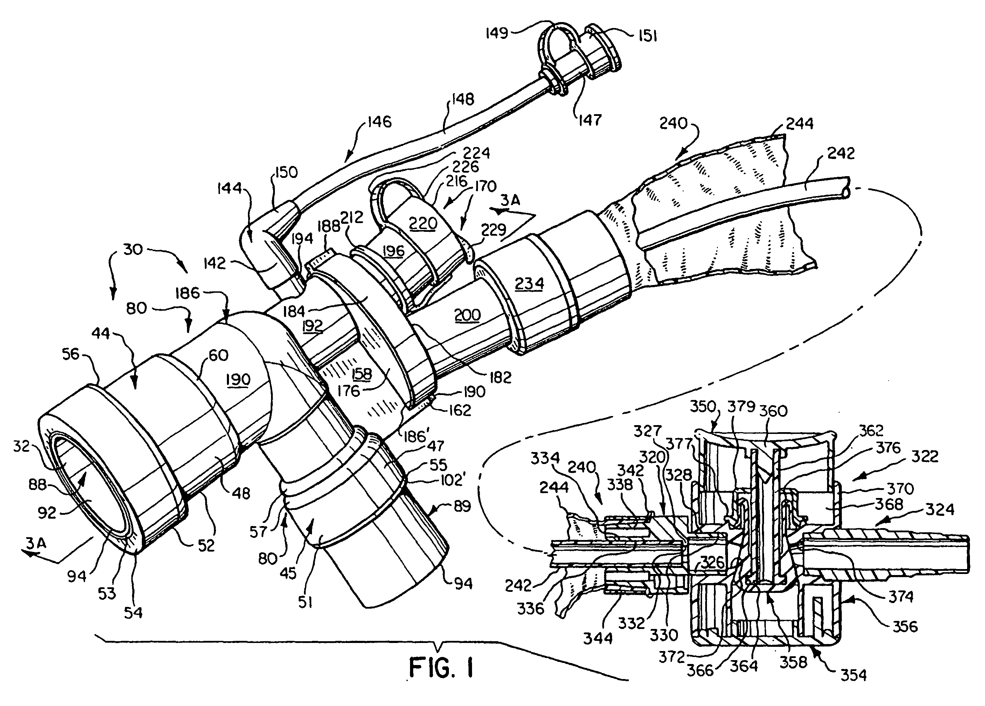

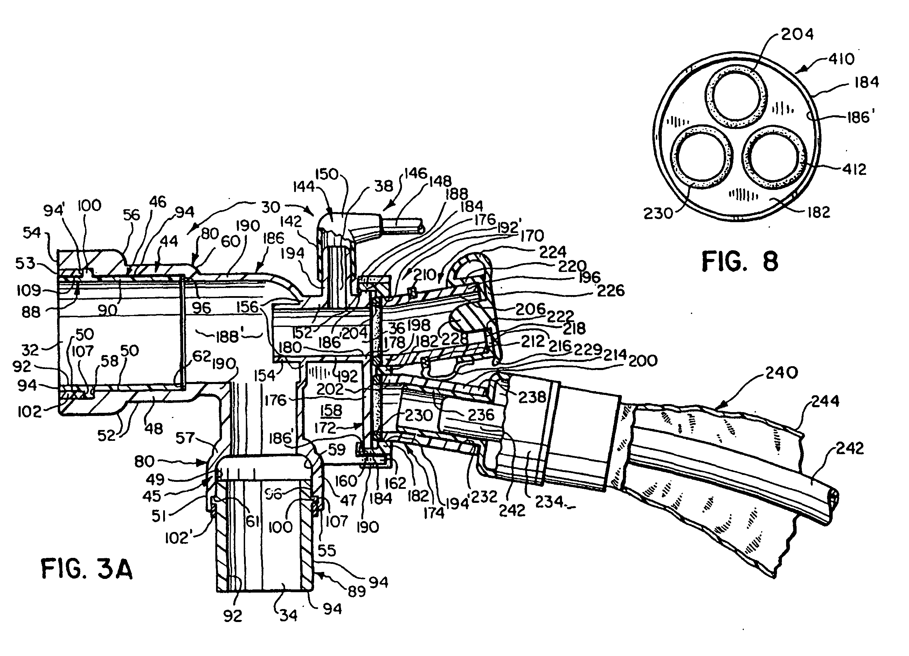

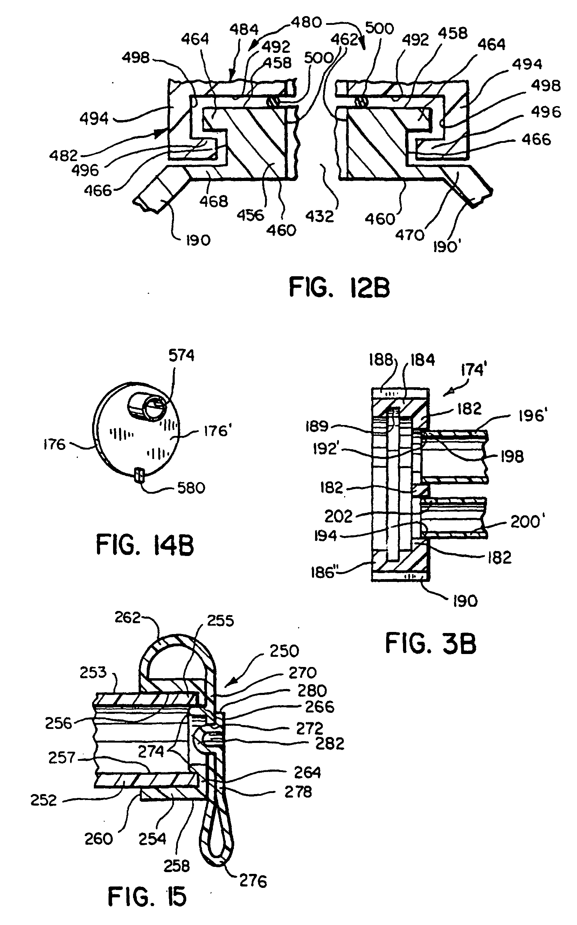

Reference is now made to the drawings wherein like numerals are used to designate like parts throughout. Figures 1, 2, and 3A illustrate a multi-access assembly, generally designated 30, for use in conjunction with the respiratory tract of an intubated medical patient ranging from infants to the aged. Assembly 30 comprises an elbow type connector, manifold, fitting, or adapter, generally designated 80. Adapter 80 has two swivel ends 88 and 89. The apparatus 30 comprises a single distal access port 32 and a plurality of proximal access ports 34, 36, and 38. See Figure 3.

For example only, access ports 32 and 34 accommodate continual cyclic patient ventilation, independent of implementation by the health care provider of any other patient respiratory access procedure. Access port 38 accommodates introduction of irrigation or wash liquid by which the exterior of an aspirating catheter tube, for example, is washed as the catheter tube is withdrawn following use. Port 38 also accommodates delivery of lavage to the interior of adapter 80. Access port 36 accommodates access by an accessory device, such as but not limited to selective insertion and subsequent removal of an aspirating catheter assembly, the catheter tube of which removes secretions from the lungs. Access port 36 also accommodates an oxygenation catheter assembly, the catheter tube of which is used in the lungs to replace residual carbon dioxide with oxygen, and/or entry of temperature or pressure monitoring instruments or obtaining samples of sputum or gases and/or to allow insertion of visual inspection instruments.

The apparatus 30 comprises a distal tracheal tube bell housing connector, generally designated 44, preferably formed of injection molded rigid medical grade synthetic resinous material, such as acrylic, cryolite, pebax, polypropylene, or the like. Connector 44 comprises a stepped annular wall 48.

Wall 48 comprises a thickness which is variable and stepped, defined by inside and outside surfaces 50 and 52. The distal end of wall 48 is defined by a blunt transverse annular distal edge 54, where the bell housing 44 comprises its largest outside diameter. Wall 48 comprises a first reduced diameter exterior annular rounded step or shoulder 56 and a second further reduced diameter exterior annular rounded step or shoulder 60.

Directly adjacent to shoulder 60 is an interior shoulder or stop 62, which limits the amount swivel sleeve 88 can be inserted at port 32. The interior surface 50 of wall 48 comprises an annular shoulder 58, which accommodates rotation of swivel sleeve 88 while preventing axial displacement thereof.

Adapter 80 also comprises a second bell housing, generally designated 45, in which proximal port 34 is formed and which is constructed to comprise components similar to bell housing 44, although bell housing 45 is substantially shorter in its axial length. Bell housing 45 is illustrated as being disposed at 90° to bell housing 46.

Bell housing 45 comprises an annular wall 47. Wall 47 comprises a thickness which is variable and stepped, defined by inside and outside surfaces 49 and 51. The distal end of wall 47 is defined by a blunt transverse annular distal edge 55, where the bell housing 45 comprises its largest outside diameter. Wall 47 comprises a reduced diameter exterior annular rounded step or shoulder 57. Shoulder 57 is also interiorly rounded at surface 59.

The interior surface 49 of wall 47 comprises an annular shoulder 61, which accommodates rotation of swivel sleeve 89, while also limiting the extent to which swivel sleeve 89 is capable of being axially inserted into the bell housing 45.

When fully assembled, sleeve 88 is rotatably positioned and secured within the bell housing 46. Sleeve 88 comprises a generally annular wall 90 comprising an interior surface 92, which is generally annular, but may be slightly divergently tapered from left to right, as viewed in Figures 1 through 3, to accommodate a press-fit but removable union with a proximal fitting of a tracheal tube, for example, in a manner well-known to those skilled in the art. Sleeve 88 also comprises a predetermined length between blunt edges 94 and 96. Edge 94 is radially flush with edge 54. Edge 96 is rotatably spaced a short distance from shoulder 62. Exterior surface 94 rotatably turns contiguously upon surface 50.

Surface 94 is interrupted by an outwardly-directed radially-extending retaining annular flange or boss 100. The location of flange 100 is selected to be adjacent to step or shoulder 58 to accommodate axial rotation contiguous with shoulder 58.

Sleeve 88 is retained in the position illustrated in Figure 3 by an annular rigid plastic collar 102 positioned between wall surfaces 50 and 94 and distally terminating in a radial edge 53, also including edges 54 and 94. Collar 102 is bonded to surface 50 of wall 48 in the position illustrated in Figure 3. Thus, collar 102 functions as a stationary bushing with flange 100 and surface 94' of sleeve 88 contiguously but rotatably engage the proximal edge surface 107 and inside annular surface 109 of collar 102.

Sleeve 89, which is rotatably coupled to bell housing 45, is substantially similar to sleeve 88, being rotatably placed within bell housing 45. Sleeve 89 is enumerated identical to sleeve 88, although it will be readily apparent that the sleeve orientation is reversed, the thickness of sleeve 88 is less than the thickness of the sleeve 89, sleeve 89 extends beyond bell housing 45 and the radial flange 100 is positioned closer to the edge 55.

Ventilating tubing is conventionally compression fit over the exposed surface 94 of sleeve 89. A tracheal fitting, as stated above, is inserted into the hollow interior of sleeve 88, to complete the ventilating circuit. Sleeve 89 rotates with any rotation imposed upon the connected ventilating tubing or, alternatively, retain an essentially stationary position if and when the adapter 80 is rotated, either intentionally or inadvertently, in respect to sleeves 88 and 89. Thus, twisting and consequential occluding or partial occluding of ventilating tubing is avoided.

The bell housings 44 and 45 integrally merge with an L-shaped segment, generally designated 186, to provide an L-shaped passageway through the adapter 80. The hollow interior of L-shaped segment 186 is designated 188'. The wall 190, comprising L-shaped segment 186, merges into two cylindrical wall segments 192 and 194, which respectively define proximal access ports 36 and 38.

Port 36 is sized and shaped so as to accommodate access by any of the previously mentioned accessory devices. Port 38 accommodates selective passage of irrigating liquid and/or lavage. Wall 194 is annular, diametrally relatively small and centrally hollow. Wall 194 receives, in force-fit relation, one leg 142 of an L-shaped elbow 144 of an irrigation or lavage delivery system, generally designated 146. System 146 comprises a hollow tube 148 which extends into and is secured within leg 150 of elbow 144, a proximal fitting, a proximal cap 151, and a tether 149 securing the cap against loss when it is disconnected from fitting 147.

Hollow cylindrical wall 192 merges as one piece with wall 194 at annular region 152 and comprises a cantilevered annular distal portion 154. Distal portion 154 merges as one piece with L-shaped segment 186 at an annular region 156 thereof. The hollow interior of wall 192 is unobstructed and diametrically smaller than the diameter of either port 32 or port 34, but larger than port 38, port 34 being diametrally smaller than port 32.

A planar, stop-supporting flange 158 is formed as one piece with the wall comprising L-shaped segment 186 and wall 192. Flange 158 comprises a slot 160 and a cantilevered stop bar or finger 162 adjacent to and forming one edge of slot 160.

The assembly 30 comprises a turret valve, generally designated 170. Turret valve 170 comprises a stator, generally designated 172, and a rotor, generally designated 174.

The stator 172 comprises a flat stationary plate 176, which is formed as one piece with both flange 158 and wall 192. The intersection between wall 192 and stator plate 176, at corner 178, defines the proximal opening 180 of port 36.

The rotor comprises a cup-shaped rotatable member or rotor, generally designated 174, comprising a radially-disposed disc-shaped base wall 182 and an annular, axially-directed flange 184, which merges as one piece with an annular inwardly-directed radial lip 186', which is force-fit over and is selectively rotatable in respect to stator plate 176 to create the assembly illustrated in Figures 1 and 3A. The relationship between the peripheral edge of the stator and the lip 186' comprises a guide, track or cam/cam follower. Thus, when assembled, the rotor 174 can be manually turned in respect to the stationary stator plate 176, to an extent and for purposes yet to be explained. The rotation is limited, in the illustrated embodiment, to approximately 180°. More specifically, the flange 184 merges with two outwardly-extending radially stops 188 and 190 carried at the exterior of flange 184 which are sized, shaped, and positioned so as to have a diameter which will cause engagement with stop cantilevered finger 162 when rotated into contiguous relation therewith. Thus, the stop 162 allows but limits rotation of the rotor 174 until either stop 188 or stop 190 makes contact therewith. Accordingly, when viewed from the right in Figure 3A, clockwise rotation of the stator 174 will bring the stop 190 into contact with stop finger 162, whereas counter-clockwise rotation of the rotor 174 will bring the stop 188 into contiguous contact with stop finger 162.

The rotor base plate 182 comprises two apertures 192' and 194' formed therein. A tube 196 is sized and shaped so that the distal end 198 thereof fits snugly into and is permanently secured therein by any suitable technique.

While tubes 196 and 200 are shown as being initially formed separate from the rotor base 180, they could be formed as one piece with rotor base 182 using conventional injection molding techniques.

Similarly, hollow tube 200, at the distal end 202 thereof is inserted snugly into aperture 194 and is secured in the inserted position using any suitable technique.

An O-ring 204 is compressively positioned between the distal end 198 of tube 196 and stator base plate 176 so as to prevent leakage at that location. The O-ring 204 has a diametral size slightly greater than the diametral size of port 36 and the diametral size of the hollow interior 206 of cylindrical wall 196. Thus, when the rotor 176 is positioned as illustrated in Figure 3A, the hollow interior 206, the hollow center of O-ring 204, and port 36 are substantially aligned.

Tube 196 has first and second legs which are slightly angular one to another, to prevent interference. Dog-leg tube 196 also comprises an external annular groove 210 into which a radially stretched tether ring 212 is placed and thereafter contracted so that ring 212, in the assembled position, exerts a compressive force upon the base of groove 210. Tether ring 212 is connected as one piece to a tether 214, which in turn is connected as one piece to a cup-shaped cap 216. Cap 216 comprises a flat base wall 218, which merges as one piece into a side annular flange 220. Base wall 218 comprises a central aperture 222.

Flange 220 merges as one piece into a second tether 224, which connects to a plug base 226. A plug 228 connects as one piece to one site of the base 226 and extends, in the fully assembled, closed position, in a distal direction. Plug 228 comprises a tip and a shank, the diameter of which is slightly greater than the diameter of aperture 222 so that full insertion of plug 228 into aperture 222 diametrally enlarges or stretches the aperture to create a compression-fit, sealed relation.

Base 226 comprises a cantilevered tab 229 by which the plug 228 can be manually pulled or pried from the aperture 222, to accommodate subsequent insertion of an accessory device through the aperture 222. Tether 224 prevents misplacement of the plug 228.

Furthermore, cap 216 can also be manually removed from the position illustrated in Figure 3 at the proximal end of dog-leg tube 196, to accommodate placement of any desired accessory device directly into the hollow interior 206 of the dog-leg tube 196 from the proximal end thereof, without passage through aperture 222. When cap 216 has been so removed, tether 214 prevents loss of either or both the cap 216 and the plug 228.

It is to be appreciated that rotor 174 is selectively manually rotated from the position of Figure 3A to a position about 180° out of phase with the position of Figure 3A, where dog-leg tube 196 is located out of alignment with port 36 and dog-leg tube 200 is in alignment with port 36. As this rotational displacement occurs, O-ring 204, at its distal contact with flat stator plate 176 simply sealingly slides therealong from the aligned to the non-aligned position.

It is also possible for the user to rotate rotor 174 so that neither dog-leg tube 196 nor dog-leg tube 200 is aligned with port 36. During any of the non-aligned conditions, rotor plate 182, due to O-rings 204 and 230 and engagement of flange 186' and wall 184, does not allow entry of contamination, nor escape of ventilating gases, nor loss of pressure from within the interior of manifold 80.

Situated in contiguous, compressed relation distally beyond distal end 202 of dog-leg tube 200 is a second O-ring 230, compressed positioned between the distal end 202 and the proximal surface of stator base plate 176. O-ring 230 moves with dog-leg tube 200 as the rotor 174 is rotated between aligned and any non-aligned position. O-ring 230 is identical to O-ring 204 in its size, having an interior diameter slightly greater than port 36 and the diameter of dog-leg tube 200. Therefore, when dog-leg tube 200 is aligned with port 36, O-ring 230 is positioned where O-ring 204 is shown in Figure 3A and does not interfere with passage of any item through tube 200 and port 36. O-rings 204 and 230 thus seal their respective dog-leg tubes 196 and 200 at their respective distal ends at all times against effluent or influent fluid flow.

Dog-leg tube 200 has a hollow interior 232, into which the distal end of certain accessory devices can be inserted. More specifically in reference to Figure 3A, a distal fitting 234 at reduced diameter male projection 236 is illustrated as being press-fit into hollow 232 of dog-leg tube 200. Insertion of end 236 is through the proximal region 238. The press-fit relationship can be manually overcome when desired. Distal fitting 234 is illustrated as being part of an aspirating catheter assembly, generally designated 240, which also comprises an aspirating catheter tube 242 around which a collapsible sheath or envelope 244 is positioned.

When dog-leg tube 200 is caused to be aligned with port 36, in the manner explained above, the health care provider can thereafter conventionally insert catheter tube 242 into the respiratory tract of the patient to remove secretions and the like in a well-known fashion. While aspirating catheter assembly 240 is illustrated as being an accessory device positioned in dog-leg tube 200, it is to be appreciated that accessory devices other than an aspirating catheter assembly can be used in conjunction with dog-leg tube 200,.

Figure 3B illustrates a rotor, generally designated 174', which varies somewhat from the rotor 174 of Figure 3A. Those parts of rotor 174' which are respectively the same as those of rotor 174 have been identically enumerated and no further description thereof is needed for one skilled in the art. Lip 186' has been eliminated and replaced by annular end 186" and blind annular groove 189, into which the peripheral edge of 176 of the stator 172 is snap-fit to create a guide or track relationship for aligned rotation of the rotor 174' in respect to the stator. Also the dog-leg tubes 196 and 200 have been replaced by straight tubes 196' and 200'.

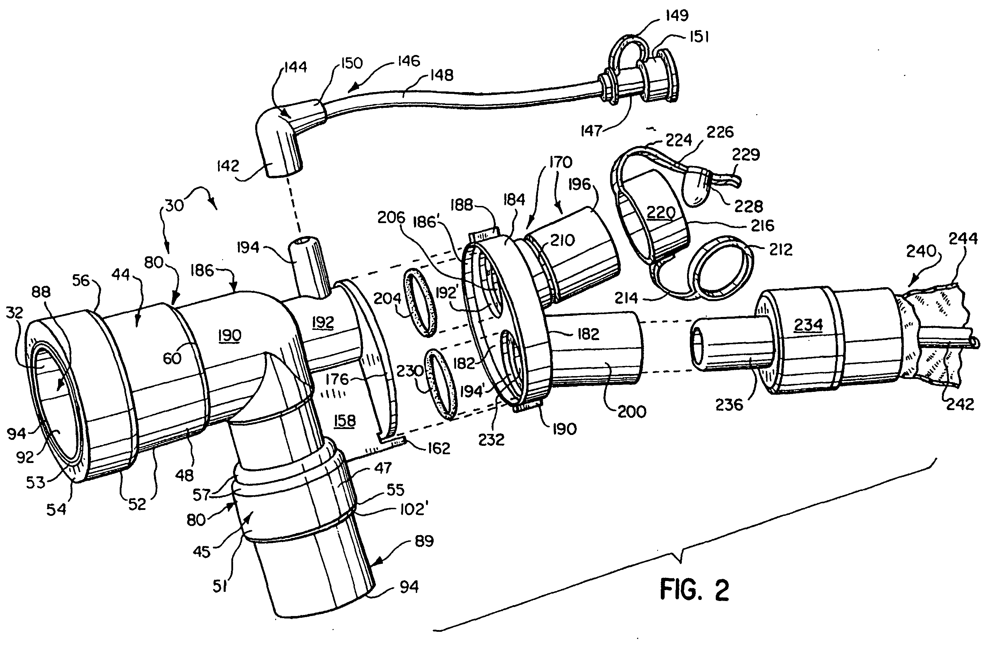

Returning to Figure 1, the previously mentioned aspirating catheter cartridge or assembly 240 comprises a proximal end which comprises seriatim a proximal fitting 320 disposed at the end of the collapsible sleeve or envelope 244, a normally closed suction valve, generally designated 322, and an exteriorally stepped tube, generally designated 324. Fitting 320 is illustrated as being formed as one piece from suitable synthetic resinous material and comprises a trailing or proximal collar 326, the exterior annular surface 328 of which is substantially the same diameter as the diameter of bore 330 forming a part of valve 322. The collar 326 is secured in the position illustrated in Figure 2 in any suitable way, such as by plastic welding, bonding, or any other suitable fashion.

The hollow interior of collar 326, at radially-directed wall 327 thereof, defines an aperture 332. Fitting 320 also comprises an annular distally-extending interior flange 334, which defines a hollow interior shown as having a uniform diameter extending to aperture 332, into which the trailing end 336 of catheter tube 242 is inserted and secured suitably in the installed position by an appropriate bonding agent, plastic welding, or in any other suitable fashion.

Fitting 320 comprises an exterior, distally-directed flange 338, which is radially spaced from flange 334. The trailing end 342 of the collapsible sheath 244 is contiguously placed over the exterior surface of flange 338, over which a collar 344 is force-fit to retain the end 342 in the assembled position.

When the normally closed valve 322 is manually depressed, negative pressure or suction is delivered from a suitable source along a suction tube to the hollow interior of fitting 324 passes across valve 322, through hollow passageways therein, through aperture 332, and along the hollow interior of tube 242 when the distal end of the tube 242 is suitably positioned within a selected lung of the patient. As a consequence, secretions accumulated in the lung are suctioned along the hollow interior of the tube 242 across aperture 332, the hollow interior of the control valve 322 and thence through stepped tube 324.

The control valve 322 comprises a manually actuated reciprocable plunger, generally designated 350, a base plate 354, a female housing member generally designated 356, and a single element elastomeric member, generally designated 358.

Plunger 350 comprises an oval-shaped exposed actuator 360, integrally connected to a rigid, vertically-oriented hollow tube 362, a flange end 364 which is seated in a correspondingly-shaped recess 366 within the hollow interior of the single element 358. Female receptacle 356 comprises a distal passageway 372 and a proximal passageway 374, which communicate one with the other across the single element 358 when the actuator 360 is depressed, by reason of the tear-shaped configuration of the single element 358. Element 358 comprises a 360° reverse bend 376, an annular flange 377, and a pear-shaped lower region, the diameter of which varies so that in the up position, the single element 358 seals passageways 372 and 374 preventing delivery of suction to the interior of the catheter tube 242. Flange 377 is anchored in the assembled position by a retainer 379 which is bonded in the position illustrated in Figure 2.

In the down position, communication of negative pressure between passageways 372 and 374 occurs around a reduced diameter part of the teardrop portion of the element 358. Element 358 also serves to inhibit introduction of atmospheric air into the valve because base plate 354 is sealed in position. Element 358 also serves as its own spring, since the element 358 is stretched in a downward direction as the actuator 360 is depressed. Consequently, the memory of the element 358 causes the single element 358 to be returned to the sealed position illustrated in Figure 2 when manual force on actuator 360 is released.

Stepped tube 324 comprises exterior annular shoulders upon which medical grade tube may be inserted and retained. Stepped tube 324 defines an interior bore in communication with bore 374 along which negative pressure is communicated selectively, as described above.

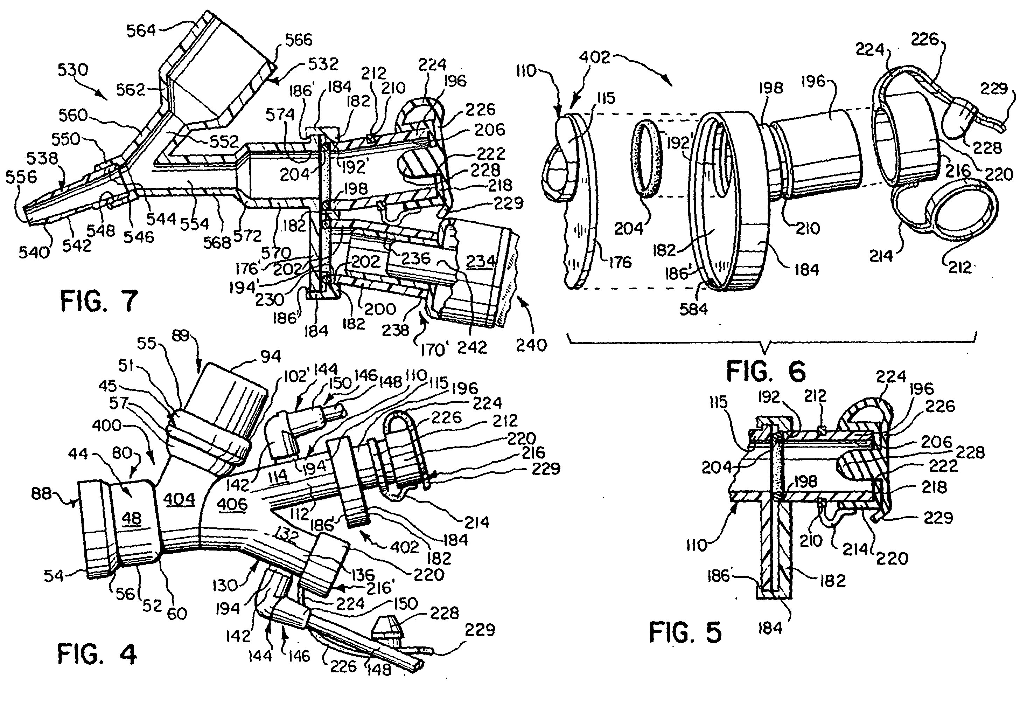

Reference is now made to Figure 7, which illustrates an additional multi-access respiratory apparatus, generally designated 530, embodying principles of the present invention. Apparatus 530 comprises extension 538, a Y-adapter, generally designated 532, an access control device 170' and accessory device 240.

Adapter 532 is illustrated as being formed of two separate parts, i.e., distal hollow tubular tip member 538, adapted to be force-fit into a port-defining opening in a tracheal fitting or into a fittingless exposed proximal end of an indwelling tracheal tube, which also accommodates patient ventilation. The tapered tip 540 accommodates such insertion, while exterior cylindrical wall surface 542 accommodates a compression fit. The proximal end 544 at exterior surface 546 is enlarged so as to snugly be received telescopically in a cylindrically stepped female receptacle 548 at distal end 550 of adapter 532. This union could also be bonded or the like, if desired.

Two proximal access passageways 552 and 554 of Y-adapter 532 merge into distal passageway 556. Passageway 552 is defined by a bell-shaped housing comprising a relatively small distal annular wall 560, a tapered conical transitional wall section 562, and an enlarged proximal cylindrically-shaped wall portion 564 terminating in a transverse blunt proximal edge 566. The other leg, which defines passageway 554, comprises a reduced diameter distal cylindrical wall portion 568, which merges distally with both wall segment 560 and wall segment 550. Cylindrical or annular wall 568 also merges proximally with a diagonally disposed stepped conically-shaped wall portion or angular shoulder 572 which in turn merges with an enlarged cylindrical segment 570.

The diameter of cylindrical segment 568 comprises a mean diameter substantially similar to the mean diameter of wall segment 560, while wall segment 564 is illustrated as comprising a mean diameter substantially greater than the mean diameter of proximal wall segment 570. However, selection of sizes may be varied in any desired way. Wall segment 564 is adapted to accommodate introduction and exhaustion of ventilating gases. The proximal port defined by wall 570 is adapted to accommodate access to the interior of Y-adapter 532 by any one of several accessory devices.

Thus, the interior surface of enlarged cylindrical or annular wall 570, at surface 574, is illustrated as sized to accommodate access of an accessory device. Barrel 570 merges as one piece with a stator plate 176' forming a part of a control device 170'. Stator plate 176' is substantially identical to previously described stator plate 176. Control device 170' is substantially identical to previously described turret valve 170, except stops 188 and 190 have been eliminated. The components of control device 170' have otherwise been enumerated identical to the above-described components of valve 170 to indicate identity or substantial identity and no further description of these components is required for those skilled in the art.

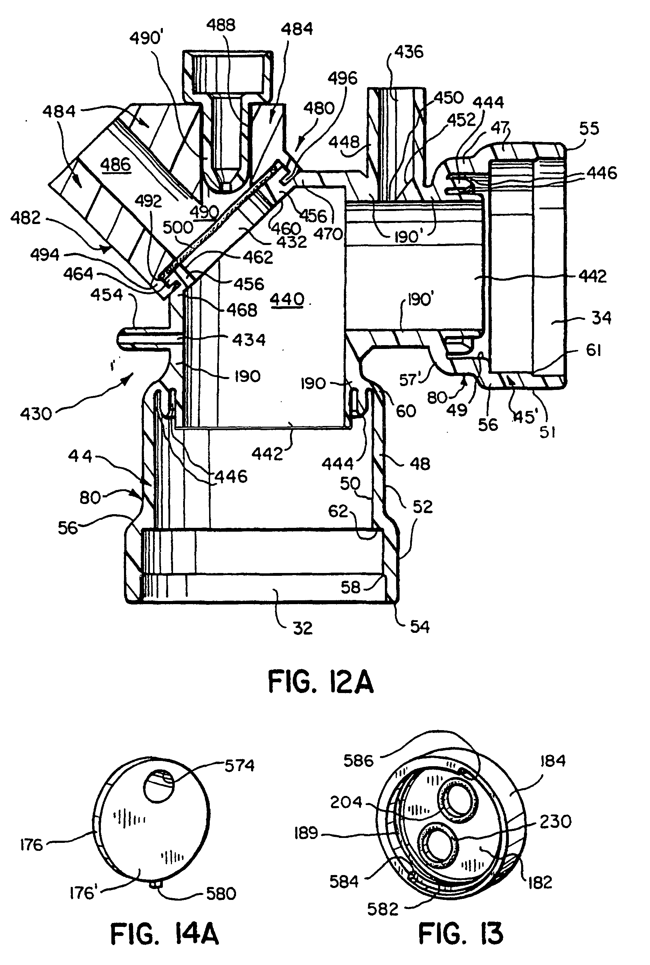

In lieu of stops 162, 188 and 190 of turret valve 170, the embodiment of Figure 7 preferably uses stops internally located within the control device 170'. The internal stops depicted in Figures 13, 14A, and 14B are suitable. More specifically, stator plate 176' may comprise a male edge stop projection 580, which is eccentric to the plate 176' and is placed in a female 180° slot or groove 582 of the flange 184 of the rotor 170'. Each end of the groove 582 comprises a blunt abutment surface or stop shoulder 584 and 586. Either stop 584 or 586 can, by rotation of flange 184 be placed contiguous with the male stop 580 to align the interior of either dog-leg tube 196 or dog-leg tube 200 with the passageway defined by surface 574. The peripheral edge 176 of plate 176' is snap-fit into blind guide groove 189, to provide aligned rotor rotation as explained above in conjunction with Figure 3B.

Reference is now made to Figures 4 through 6, which illustrate a further multi-access manifold, fitting, or adapter, generally designated 400 assembled with a control device, generally designate 402, which is in the nature of a turret valve having only one passageway through the rotor thereof for alignment and misalignment with a proximal access port of the manifold 400 to accommodate access to the interior of the manifold 400 by an accessory device through the single-passageway defining control device 402.

Hollow manifold 400 comprises the previously described distal hollow connector 44 for connection to the proximal end of an indwelling tracheal tube. Manifold 400 also comprises the previously described proximal ventilating hollow connector 89, connected to bell housing 45, except bell housing 45 in Figure 4 is disposed at an acute angle to connector 44 in lieu of the 90° angle depicted in Figures 1-3. Manifold 400 comprises two hollow bifurcated segments 404 and 406. Manifold 400 further comprises an angularly-disposed proximally-directed barrel, generally designated 110. Proximally-directed barrel 110 comprises a cylindrical hollow wall 112, the exterior surface 114 of which is annular. The hollow interior of wall 112 may be slightly tapered divergently in a distal direction to receive, in compression-fit relation, a distal fitting of certain accessory devices.

Bell housing 45 and barrel 110 merge with each other at bifurcated segment 404. The acute angle between the two, illustrated as being on the order of about 45°, accommodates ease of internal displacement of certain accessory devices through the barrel 110, the bifurcated segment 404 and the connector 44.

Control device 402 is carried at the proximal end 115 of barrel 110. Control device 402 comprises a turret valve, certain components of which are the same, and so numbered in Figures 4-6, as previously described in respect to turret valve 170 except as otherwise specified herein. O-ring 230, aperture 194, dog-leg tube 200, and the external stops 188, 190, and 162 have been eliminated. Internal stops 584 and 586 are used. Thus, turret valve 402 provides only a single or registered enabled position, i.e., where dog-leg tube 196 thereof is substantially aligned with port 36.

Barrel 114 is illustrated as being equipped with the previously described lavage/irrigation delivery system 146.

Manifold 400 further comprises a second angularly-disposed proximally-directed hollow barrel, generally designated 130. Barrel 130 comprises an exterior substantially cylindrical hollow wall 136, the interior of which is hollow and may be slightly divergently tapered in a distal direction. Barrel 130 comprises an exterior surface 132 and terminates in a proximal end which telescopically receives cap 216'. Barrel 130 is disposed at a small acute angle in respect to the axis of connector 44 which may be on the order of about 30°, as illustrated in Figure 4, although other angles could be used. The use of small acute angles for the proximal passageways or ports functions to facilely center the distal end of an inserted accessory device, such as a catheter tube, as it is advanced.

The proximal end of barrel 130 is illustrated as receiving cap 216', which is identical to cap 216, except ring 212 and tether 24 have been eliminated.

Barrel 130 merges with barrel 110 at bifurcated segment 406 and is illustrated as being equipped with a lavage/irrigation delivery system 146.

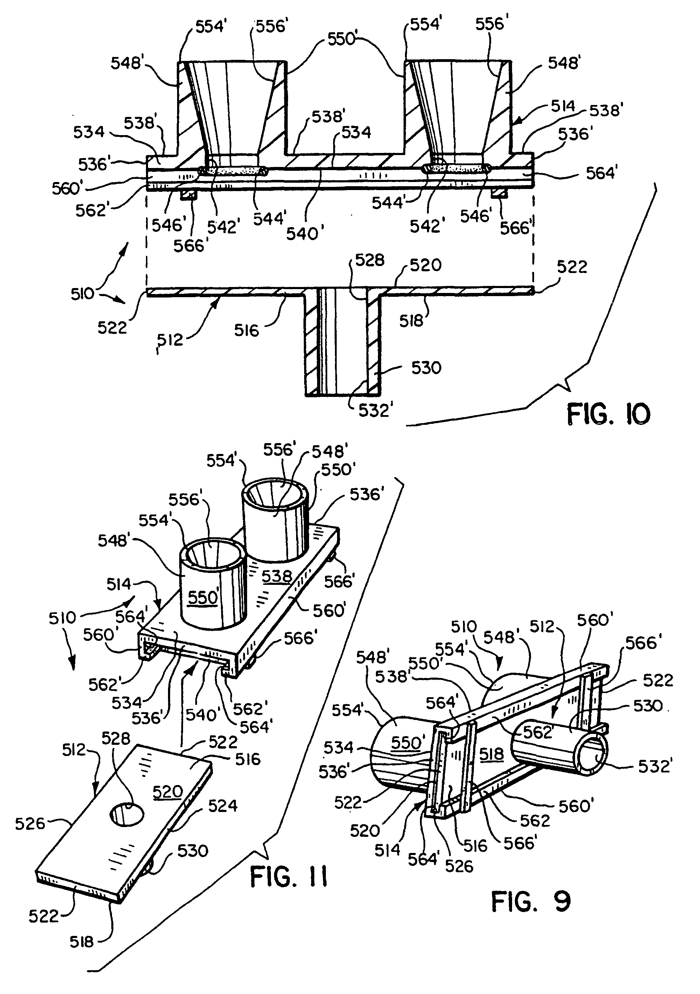

The present invention embraces use of a control device in conjunction with a connector, manifold, fitting and/or adapter in which the control device is not limited to one or two selective repositionable passageways. For example, three such passageways may be used as is illustrated in Figure 8. Figure 8 depicts only a rotor portion, generally designated 410, of a turret valve control device, which also comprises a stator plate of the type heretofore described. Rotor 410 comprises the previously described plate or base wall 182, flange 184, and lip 186'. Plate 182 of Figure 8 comprises three ports or passageways around which O-ring seals 204, 230, and 412 are respectively compressively situated for the same purpose described above.

By manually rotating rotor 410 in respect to its associated stationary stator plate, any desired one of the three passageways can be enabled or placed in registry with a proximal port of an associated connector, fitting, manifold or adapter, while at the same time the other two passageways are disabled. Any such enabled passageway accommodates communication into the manifold, fitting or adapter by a desired accessory device. Any passageway in a disabled or non-registered position can accommodate attachment and detachment of a desired accessory device at that site.

Reference is now made to Figures 12A and 12B which illustrate a further manifold, generally designated 430, which is in the form of an elbow fitting. The features and components of elbow 430 duplicates a substantial number of the features and components of previously described elbow 186 to the extent Figure 12 is correspondingly enumerated.

Elbow 430 differs from elbow 186 in the ways described below. Passageway- defining walls 192 and 194 and port 36 have been replaced by port 432 and wash/lavage port 434. Swivel sleeves for ports 32 and 34 are not shown for ease of illustration and description.

The interior configuration adjacent to shoulders 60 and 57' for bell housings 44 and 45' has been altered. Bell housing 45' has been provided with a further medication port 436. Bell housing 45' in Figure 12A comprises two steps or shoulders 56 and 57', so that bell housing 45', while shorter than bell housing 44, is internally and externally structurally and functionally substantially the same.

The hollow L-shaped corner 440 of elbow fitting 430 is defined primarily by two generally cylindrical or annular walls 190 and 190'. Walls 190 and 190' each comprise an annular segment 442 which cantilevers into ports 32 and 34, respectively. At longitudinally-directed cantilevered ring 444 is disposed in concentric relationship between wall 48 and the adjacent segment 442 and between wall 57 and the adjacent segment 442. Thus, two spaced parallel annular blind grooves 446 are formed on opposite sides of each ring 444. These blind grooves 446 are designed to receive spaced sealing elements of a swivel sleeve when inserted into each of ports 32 and 34, respectively.

Wall 190' merges as one piece with radially-directed annular wall 448, the hollow interior of which comprises port 436. Port 436 opens into the hollow interior of the L-shaped corner 440 at passage 450, adjacent to diagonal baffle 452, by which mediation delivered to port 436 is deflected toward the hollow interior of L-shaped corner 440 to enhance delivery, in an atomized form, to the patient, using ventilating gases as a carrier.

Wall 190 merges as one piece with radially-directed annular wall 454, the hollow interior of which comprises port 434. Lavage and/or irrigation liquid can selectively be delivered through port 434, for example, in the manner described above. Where port 436 is used for medication, use of port 434 can be limited to irrigation, if desired.

Walls 190 and 190' merge at the outside portion of L-shaped corner 440 as one piece with a diagonally disposed hollow flange 456.

Flange 456, as best seen in Figure 12B, is generally planar and of uniform thickness, except to the extent otherwise indicated herein. Flange 456 comprises a flat surface 458, an opposite parallel flat surface 460 and an annular or circular aperture-forming edge wall 462 which defines a hollow circular opening in flange 456 comprising port 432.

Flange 456 also comprises an annular ring 464 parallel and adjacent to an annular blind groove 466 comprising parallel side walls and a perpendicular base. Flange 456 merges as one piece with wall 190 at bridge 468 and with wall 190' at bridge 470. Functionally flange 456 comprises a stator plate, of still another access control device, with central opening. This control device is generally designated 480.

In addition to stator flange 456, control device 480 comprises a rotor, generally designated 482. Rotor 482 comprises a body 484 of material which defines angularly disposed ports 486 and 488. Ports 486 and 488 merge at bifurcated segment 490, which is at all times aligned with and has essentially the same diametral size as port 432. By manually rotating rotor 482, either port 488 or port 486 is principally placed in registry with port 32. The registry position of port 488 is shown in Figure 12A.

An accessory device can be received at either or both ports 486 and 488, a distal fitting 490 of an accessory device being illustrated in Figure 12A as compression-fit into port 488, for illustrative purposes. Either or both ports 486 and 488 can be temporarily plugged, capped, or closed in any suitable way, such as is explained above.

Rotor body 484 comprises a radially disposed somewhat annular flat distal surface 492, which is parallel to but spaced from surface 458. Body 484 comprises an annular distal extension wall 494 and an inwardly directed annular radial flange 496, which connects to wall 494 and is parallel to surface 492. The space between wall 496 and surface 492 comprises an annular groove 498 comprising parallel side wall surfaces and a perpendicular base surface into which flange 464 stationarily extends in spaced but parallel relation.

O-ring 500 is compressively interposed between surfaces 458 and 492 slightly beyond the diametral limits of port 432 and hollow bifurcation 490. The O-ring seal 500 accommodates rotation of rotor 482 to align either port 486 or port 488 with port 32, while preserving its sealing relation with surfaces 458 and 492.

The inter-relationships between the U-shaped annular groove comprising surface 498 and annular flange 464 and the U-shaped annular groove comprising surface 466 and annular flange 496 comprise guides or tracks, which maintain alignment during the rotation of rotor 482.

Reference is now made to Figures 9-11 which illustrate a further access control device, generally designated 510. Access control device 510 comprises a slider valve by which any one of a plurality of proximal ports can be enabled, i.e., aligned or placed in registry with a single distal port. Slider valve 510 comprises stator element, generally designated 512, and a slider element, generally designated 514.

Stator 512 comprises a flat elongated plate 516, illustrated as being of uniform thickness throughout, extending between a distal surface 518 and a proximal surface 520. Plate 516 also comprises two parallel side edges 522 and parallel bottom and top edges 524 and 526.

Single port stator plate 516 is centrally interrupted by a single circular aperture 528. Plate 516 merges as one piece with a distal cantilever tube 530, the cylindrical hollow interior 532' of which is aligned with and of the same diametral size as aperture 528. Tube 530 is adapted to be compression-fit to a proximal access port of a connector, manifold, fitting, or adapter.

Dual port slider 514 comprises a flat rectangular plate segment 534, which comprises two parallel edges 536', a proximal surface 538', and a distal surface 540'. Plate 534 is illustrated as being of uniform thickness. Spaced openings 542' exist through plate 534. A rounded seat 544' is provided in surface 540' of wall 534 around each opening 542' to receive an O-ring 546'.

A generally cylindrical interiorly hollow wall 548' surrounds each opening 542' at and extending from surface 538'. Each generally cylindrical wall 548' comprises a cylindrical surface 550', a blunt annular edge 554', and a divergently tapered truncated cone-shaped hollow interior 556.

The two tapered interiors 556' respectively accommodate compression-fit insertion and removal of a distal fitting of an accessory device. Hollow interior 556' can be closed, capped, plugged, or the like when not in use.

The plate 534 merges as one piece, top and bottom, with a channel-shaped edge comprising a transversely-directed linear edge wall 560' connected to the top of the plate, at a 90° angle thereto, and an inwardly-directed linear lip or flange 562' connected to the proximal portion of the wall 560'. Since both flanges 562' are parallel to but spaced from plate 534, a U-shaped groove 564' is formed top and bottom between each flange 562' and plate 534. The size of each vertically aligned groove 564' is substantially the same. The width of the grooves 564' is just slightly greater than the thickness of plate 516 and the vertical distance between top and bottom walls 560' is slightly greater than the vertical dimension of plate 516 so that when plate 516 is placed within the grooves 564' slider 514 can be displaced back and forth within the grooves 564, which serve as tracks or guides for such displacement.

When assembled, each O-ring 544' is compressed between the associated seat 546' and surface 520 of plate 516 to seal the associated port 542'. Both O-rings 544' slide back and forth with slider 514, while retaining their respective sealed relation between seats 546' and surface 520.

A pair of bars 566' if desired, can be integrally connected to and span between the top and bottom flanges 562' at spaced locations such that contact between one bar 566' and the port wall 530 will align one port 542' with port 532, while contact between the other bar 566' and the port wall 530 will align the other port 542' with port 532. Thus, either port 542' can be enabled or placed in registry for communication of an accessory device to the interior of an associated manifold, for example, via aligned ports 542' and 532. The non-aligned port 542' is disabled, allowing for attachment and/or detachment of a selected accessory device at the disabled port 542'.

Reference is now made to Figure 15 which illustrates an access control appliance, generally designated 250, which is illustrated as being fitted upon the proximal end of an access tube 252, similar in type and purpose to the previously described tubes 196, 200, 196', and 200'. The access control appliance 250 is in the nature of a plug for the proximal end of an access port forming a part of an access control device of the type described above. Plug 250 accommodates access of a variety of accessory devices, the distal ends of which may comprise differing diameters.

Specifically, plug 250 comprises a collar 254, which is annular in configuration, comprising a wall illustrated as being of uniform thickness comprising an interior annular surface 256 and an exterior annular surface 258. Preferably, the unstressed diameter of surface 256 is slightly less than the outside diameter, at surface 253 of access tube 252. Thus, collar 254, formed of suitable synthetic or elastomeric material is stretched to accommodate placement, as illustrated in Figure 15, over the proximal end 255 of the access tube 252 so that when the stretching force is removed, collar 254 is retained by a memory-derived compressive force radially exerted upon access tube end 255.

The blunt distal edge 260 of the collar 254 is interrupted by a thin tether 262, formed as one piece with collar 254.

Collar 254 merges with an annular inwardly-directed flange 264, which has a hollow central opening defined by flange edge 266. The unstressed diameter of the opening at edge surface 266 is somewhat smaller than the interior diameter at wall surface 257 of the access tube 252.

It is to be appreciated that collar 254 can, if desired, be tethered to the wall 252 in any suitable fashion such as by use of the previously described tether strap 214 and tether ring 212.

By complete removal of the cap 254 from the proximal end 255 of the access tube 252, the distal portion of an access device having a maximum diameter can be accommodated, either by press-fit insertion within the hollow interior of the access tube 254, or by engagement at exterior surface 253 of tube 252. An accessory device intended to be inserted within tube 252, but having a diameter at the distal end thereof somewhat less than the diameter of surface 257 can be accommodated by removal of all encumbrances within the opening formed by edge 266, followed by force-fit insertion of the somewhat diametrally smaller distal portion of accessory device into the hollow interior of tube 252 so as to create a compression fit at edge 266.

Tether 262 merges with a linear base 270. The base 270 is interrupted by a relatively small central aperture defined by edge surface 272. A distally extending boss 274 is concentrically disposed around aperture defining wall edge 272. The outside diameter of the boss 274 is selected, preferably so as to be slightly larger than the unstressed diameter of the aperture defined by edge surface 266. Thus, when the boss 274 is inserted into the aperture defined by edge surface 266, the diameter at edge 266 is slightly increased and a compression fit between edge 266 and the outside surface of boss 274 is created which tends to hold the boss 274 in the inserted position illustrated in Figure 15 against inadvertent removal. The aperture formed by edge 272, has an unstressed diameter typically smaller than the smallest diameter of the distal end of an accessory device for which access is needed to the hollow interior of tube 252. Thus, when the aperture defined by edge surface 272 is fully open, the smallest diametral distal fitting of an accessory device is inserted therethrough into the hollow interior of tube 252 in such a fashion as to create a compression fit between the distal portion of the accessory device and the edge surface 272.

The base 270 merges as one piece with an additional tether band 276. Tether band 276 merges as one piece with a further base 278, which terminates at base edge 280. Base 278 merges with a hollow grounded distally-extending tip 282, the outside diameter of which immediately adjacent base 278 is preferably, when unstressed, somewhat larger than the unstressed diameter of aperture-defining edge 272. Accordingly, when tip 282 is inserted through aperture-defining edge 272, the diameter of edge 272 is slightly enlarged resulting in a compression fit relationship between edge 272 and tip 282.

Based upon the foregoing, removal of tip plug 282 from aperture-defining edge surface 272 accommodates insertion of a relatively small distal end of one accessory device. Removal of the boss 274 from the aperture formed by edge surface 266 accommodates insertion of a somewhat diametrally larger distal portion of another accessory device. Removal of cap 250 in its entirety accommodates insertion of a still diametrally larger distal portion of a third accessory device.

The present embodiments therefore to be considered in all respects as illustrative and are not restrictive, the scope of the invention being indicated by the appended claims rather than by the foregoing description, and all changes which come within the meaning and range of equivalency of the claims are therefore intended to be embraced therein.

## Claims

1. An adaptor assembly (30) for providing ventilation of an intubated patient's respiratory tract, and for enabling access to the tract for respiratory therapy without interruption of ventilation, the adaptor assembly comprising;
a hollow adaptor (80) comprising a distal port (32) for connection to the proximal end of an endotracheal tube, a first proximal port (34) for connection to a ventilator and at least one non-ventilating proximal port (36);
the assembly further comprising access control means (170, 171, 402, 410, 480, 514) for selectively locating a first access port (192', 488, 542') in a position of alignment with said non-ventilating proximal port to provide access to the interior of the adaptor through said first access port and the non-ventilating proximal port by an accessory device; the access control means comprising a stationary part (172) comprising a stator plate (176) having an opening there through communicating into said non-ventilating proximal port and a movable part (174) having the access port whereby the access port is movable away from said position of alignment; and **characterized in that** at least one compressible seal (204) is provided between said stationary part and said movable part of the access control means such seal being arranged to surround at least the first access port, and the movable part of said access control means further comprises a peripheral flange (184) having an inwardly directed lip (186') for receiving said stator plate to secure the movable part to the stationary part of the access control means.

2. An adaptor as claimed in claim 1 wherein the access port is movable to a position in which it is sealed from the non-ventilating proximal port.

3. An adaptor assembly as claimed in claim 2 wherein said seal (204) is displaceable in use with the movable part of the access control means, whilst remaining in engagement between the movable and stationary parts.

4. An adaptor assembly as claimed in any preceding claim wherein said moveable part further comprises a second access port (200, 194').

5. An adaptor assembly as claimed in claim 4 wherein the first and second access port are each encircled by a compressible seal (204, 230').

6. An adaptor assembly as claimed in claim 4 or 5 wherein said movable part is movable between a first position wherein the first access port is in alignment with the opening of said stationary part and said second access port is not in alignment with said opening, and a second position wherein said second access port is in alignment with said opening and said first access port is not in alignment with said opening.

7. An adaptor assembly as claimed in claim 6 wherein said movable part is movable to a third position wherein neither of said first and second access ports is in alignment with said opening.

8. An adaptor assembly as claimed in any preceding claim wherein said seal comprises an O-ring (204).

9. An adaptor assembly as claimed in any preceding claim wherein said movable part is rotatable.

10. An adaptor assembly as claimed in any of the preceding claims and further comprising a second non-ventilating proximal port.

11. An adaptor assembly as claimed in any preceding claim and further comprising at least one further access port (38), for providing introduction of a lavage fluid to the interior of the adaptor in conjunction with at least one of said proximal ports.

12. An adaptor assembly as claimed in claim 11 wherein said further access port is positioned adjacent to said proximal port and essentially normal thereto.

13. The adaptor assembly of claim 12 wherein said further access port is positioned adjacent to said first non-ventilating proximal port.

14. The adaptor assembly of any preceding claim wherein said adaptor and said stationary part of the access control means are formed as one piece.

15. The adaptor assembly of any preceding claim wherein said first non-ventilating proximal port of the adaptor is in substantial axial alignment with the distal port of the adaptor.

16. An adaptor assembly as claimed in any preceding claim and in combination with a first catheter(242), connected to said first access port, for aspirating the respiratory tract when desired, said first catheter being slidably positionable through the first access port for a substantially axially aligned entry through the interior of the adaptor and into the proximal end of the endotracheal tube.

17. The combination of claim 16 wherein the distal end of said first catheter is permanently connected to the first access port.

18. A combination as claimed in claim 16 wherein the distal end of said first catheter is removably connected to said first access port.

19. The combination of any of claims 16 to 18 wherein the movable port of said adaptor includes a second access port, and the combination further comprises a second catheter, connected to said second access port, for providing other treatment including any one of delivery of fluids, administering medication, monitoring, removing patient samples or providing visual inspection of the respiratory tract when desired, the second catheter being slidably positionable through the second access port for substantially axially aligned entry through the interior of the adaptor and into the proximal end of the endotracheal tube.

20. The combination of claim 19 wherein the distal end of said second catheter is permanently connected to said second access port.

21. The combination of claim 19 wherein the distal end of said second catheter is removably connected to said second access port.

## Patentansprüche

1. Adapterbaugruppe (30), die Belüftung des Respirationstraktes eines intubierten Patienten gewährleistet und Zugang zu dem Trakt zur Respirationstherapie ohne Unterbrechung der Belüftung ermöglicht, wobei die Adapterbaugruppe umfasst:
einen hohlen Adapter (80), der einen vorderen Anschluss (32) zur Verbindung mit dem hinteren Ende eines Endotrachealtubus, einen ersten hinteren Anschluss (34) zur Verbindung mit einer Belüftungseinrichtung, und wenigstens einen nicht belüftenden hinteren Anschluss (36) umfasst;
wobei die Baugruppe des Weiteren eine Zugangs-Steuereinrichtung (170, 171, 402, 410, 480, 514) zum selektiven Positionieren eines ersten Zugangsanschlusses (192', 488, 542') in einer Position der Ausrichtung auf den nicht belüftenden hinteren Anschluss umfasst, um mit einer Zusatzvorrichtung Zugang zum Inneren des Adapters über den ersten Zugangsanschluss und den nicht belüftenden hinteren Anschluss zu erzeugen; wobei die Zugangs-Steuereinrichtung einen stationären Teil (172), der eine Statorplatte (176) mit einer Öffnung durch diese hindurch, die mit dem nicht belüftenden hinteren Anschluss in Verbindung steht, und einen beweglichen Teil (174) umfasst, der den Zugangsanschluss aufweist, so dass der Zugangsanschluss aus der Position der Ausrichtung wegbewegt werden kann; **dadurch gekennzeichnet, dass** wenigstens eine zusammendrückbare Dichtung (204) zwischen dem stationären Teil und dem beweglichen Teil der Zugangs-Steuereinrichtung vorhanden ist, wobei die Dichtung so eingerichtet ist, dass sie wenigstens den ersten Zugangsanschluss umgibt, und der bewegliche Teil der Zugangs-Steuereinrichtung des Weiteren einen Umfangsflansch (184) mit einer nach innen gerichteten Lippe (186') zur Aufnahme der Stator-Platte umfasst, um den beweglichen Teil an dem stationären Teil der Zugangs-Steuereinrichtung zu befestigen.

2. Adapter nach Anspruch 1, wobei der Zugangsanschluss an eine Position bewegt werden kann, an der er gegenüber dem nicht belüftenden hinteren Anschluss abgedichtet ist.

3. Adapterbaugruppe nach Anspruch 2, wobei die Dichtung (204) in Funktion mit dem beweglichen Teil der Zugangs-Steuereinrichtung verschoben werden kann, während sie in Eingriff mit dem beweglichen und dem stationären Teil bleibt.

4. Adapterbaugruppe nach einem der vorangehenden Ansprüche, wobei der bewegliche Teil des Weiteren einen zweiten Zugangsanschluss (200, 194') umfasst.

5. Adapterbaugruppe nach Anspruch 4, wobei der erste und der zweite Zugangsanschluss jeweils von einer zusammendrückbaren Dichtung (204, 230') umschlossen sind.

6. Adapterbaugruppe nach Anspruch 4 oder 5, wobei der bewegliche Teil zwischen einer ersten Position, in der der erste Zugangsanschluss auf die Öffnung des stationären Teils ausgerichtet ist und der zweite Zugangsanschluss nicht auf die Öffnung ausgerichtet ist, und einer zweiten Position bewegt werden kann, in der der zweite Zugangsanschluss auf die Öffnung ausgerichtet ist und der erste Zugangsanschluss nicht auf die Öffnung ausgerichtet ist.

7. Adapterbaugruppe nach Anspruch 6, wobei der bewegliche Teil an eine dritte Position bewegt werden kann, an der weder der erste noch der zweite Zugangsanschluss auf die Öffnung ausgerichtet ist.

8. Adapterbaugruppe nach einem der vorangehenden Ansprüche, wobei die Dichtung einen O-Ring (204) umfasst.

9. Adapterbaugruppe nach einem der vorangehenden Ansprüche, wobei der bewegliche Teil gedreht werden kann.

10. Adapterbaugruppe nach einem der vorangehenden Ansprüche, der des Weiteren einen zweiten nichtbelüftenden hinteren Anschluss umfasst.

11. Adapterbaugruppe nach einem der vorangehenden Ansprüche, die des Weiteren wenigstens einen weiteren Zugangsanschluss (38) umfasst, der Einleiten einer Spülflüssigkeit in das Innere des Adapters im Zusammenhang mit wenigstens einem der hinteren Anschlüsse gewährleistet.

12. Adapterbaugruppe nach Anspruch 11, wobei der weitere Zugangsanschluss an den hinteren Anschluss angrenzend und im Wesentlichen senkrecht dazu angeordnet ist.

13. Adapterbaugruppe nach Anspruch 12, wobei der weitere Zugangsanschluss an den ersten nichtbelüftenden hinteren Anschluss angrenzend angeordnet ist.

14. Adapterbaugruppe nach einem der vorangehenden Ansprüche, wobei der Adapter und der stationäre Teil der Zugangs-Steuereinrichtung als ein Stück ausgebildet sind.

15. Adapterbaugruppe nach einem der vorangehenden Ansprüche, wobei der erste nichtbelüftende hintere Anschluss des Adapters im Wesentlichen axial auf den vorderen Anschluss des Adapters ausgerichtet ist.

16. Adapterbaugruppe nach einem der vorangehenden Ansprüche in Kombination mit einem ersten Katheter (242), der mit dem ersten Zugangsanschluss verbunden ist, um, wenn gewünscht, den Respirationstrakt zu aspirieren, wobei der erste Katheter durch den ersten Zugangsanschluss zum im Wesentlichen axial ausgerichteten Eintritt über das Innere des Adapters und in das hintere Ende des Endotrachealtubus gleitend positioniert werden kann.

17. Kombination nach Anspruch 16, wobei das vordere Ende des ersten Katheters fest mit dem ersten Zugangsanschluss verbunden ist.

18. Kombination nach Anspruch 16, wobei das vordere Ende des ersten Katheters abnehmbar mit dem ersten Zugangsanschluss verbunden ist.

19. Kombination nach einem der Ansprüche 16 bis 18, wobei der bewegliche Anschluss des Adapters einen zweiten Zugangsanschluss enthält und die Kombination des Weiteren einen zweiten Katheter umfasst, der mit dem zweiten Zugangsanschluss verbunden ist, um andere Behandlung einschließlich einer Zufuhr von Fluiden, der Verabreichung von Medikamenten, der Überwachung, der Entnahme von Patienten-Proben zu gewährleisten oder visuelle Untersuchung des Respirationstraktes zu gewährleisten, wenn dies gewünscht wird, wobei der zweite Katheter über den zweiten Zugangsanschluss zum im Wesentlichen axial ausgerichteten Eintritt über das Innere des Adapters und in das hintere Ende des Endotrachealtubus gleitend positioniert werden kann.

20. Kombination nach Anspruch 19, wobei das vordere Ende des zweiten Katheters fest mit dem zweiten Zugangsanschluss verbunden ist.

21. Kombination nach Anspruch 19, wobei das vordere Ende des zweiten Katheters abnehmbar mit dem zweiten Zugangsanschluss verbunden ist.

## Revendications

1. Ensemble d'adaptateur (30) pour assurer la ventilation des voies respiratoires d'un patient intubé, et pour permettre un accès aux voies respiratoires pour procéder à une thérapie respiratoire sans interruption de la ventilation, l'ensemble d'adaptateur comprenant:
un adaptateur creux (80) comprenant un port distal (32) à connecter au port proximal d'un tube endotrachéal, un premier port proximal (34) à connecter à un ventilateur et au moins un port proximal de non ventilation (36);
l'ensemble comprenant en outre un moyen de contrôle d'accès (170, 171, 402, 410, 480, 514) pour placer d'une façon sélective un premier port d'accès (192', 488, 542') dans une position en alignement avec ledit port proximal de non ventilation afin de fournir un accès à l'intérieur de l'adaptateur à travers ledit premier port d'accès et ledit port proximal de non ventilation par un dispositif accessoire; le moyen de contrôle d'accès comprenant une partie stationnaire (172) comprenant une plaque de stator (176) comportant une ouverture qui la traverse communiquant dans ledit port proximal de non ventilation, et une partie mobile (174) comprenant le port d'accès, dans lequel le port d'accès est mobile pour s'écarter de ladite position d'alignement; et **caractérisé en ce qu'**au moins un joint compressible (204) est prévu entre ladite partie stationnaire et ladite partie mobile du moyen de contrôle d'accès, ledit joint étant arrangé de manière à entourer au moins le premier port d'accès, et **en ce que** la partie mobile dudit moyen de contrôle d'accès comprend en outre une bride périphérique (184) comportant une lèvre dirigée vers l'intérieur (186') pour recevoir ladite plaque de stator afin de fixer la partie mobile sur la partie stationnaire du moyen de contrôle d'accès.

2. Adaptateur suivant la revendication 1, dans lequel le port d'accès est mobile jusqu'à une position dans laquelle il est étanche par rapport au port proximal de non ventilation.

3. Ensemble d'adaptateur suivant la revendication 2, dans lequel, lors de l'utilisation, ledit joint (204) peut être déplacé avec la partie mobile du moyen de contrôle d'accès, tout en restant en engagement entre les parties mobile et stationnaire.

4. Ensemble d'adaptateur suivant l'une quelconque des revendications précédentes, dans lequel ladite partie mobile comprend un deuxième port d'accès (200, 194').

5. Ensemble d'adaptateur suivant la revendication 4, dans lequel les premier et deuxième ports d'accès sont chacun encerclés par un joint compressible (204, 230').

6. Ensemble d'adaptateur suivant la revendication 4 ou 5, dans lequel ladite partie mobile est mobile entre une première position dans laquelle le premier port d'accès est en alignement avec l'ouverture de ladite partie stationnaire et où ledit deuxième port d'accès n'est pas en alignement avec ladite ouverture, et une deuxième position dans laquelle ledit deuxième port d'accès est en alignement avec ladite ouverture et où ledit premier port d'accès n'est pas en alignement avec ladite ouverture.

7. Ensemble d'adaptateur suivant la revendication 6, dans lequel ladite partie mobile est mobile jusqu'à une troisième position dans laquelle aucun des premier et deuxième ports d'accès n'est en alignement avec ladite ouverture.

8. Ensemble d'adaptateur suivant l'une quelconque des revendications précédentes, dans lequel ledit joint comprend un joint torique (204).

9. Ensemble d'adaptateur suivant l'une quelconque des revendications précédentes, dans lequel ladite partie mobile est rotative.

10. Ensemble d'adaptateur suivant l'une quelconque des revendications précédentes, et comprenant en outre un deuxième port proximal de non ventilation.

11. Ensemble d'adaptateur suivant l'une quelconque des revendications précédentes, comprenant en outre au moins un port d'accès supplémentaire (38) pour permettre l'introduction d'un fluide de lavage à l'intérieur de l'adaptateur en conjonction avec au moins un desdits ports proximaux.

12. Ensemble d'adaptateur suivant la revendication 11, dans lequel ledit port d'accès supplémentaire est positionné à proximité dudit port proximal et est essentiellement normal à celui-ci.

13. Ensemble d'adaptateur suivant la revendication 12, dans lequel ledit port d'accès supplémentaire est positionné à proximité dudit premier port proximal de non ventilation.

14. Ensemble d'adaptateur suivant l'une quelconque des revendications précédentes, dans lequel ledit adaptateur et ladite partie stationnaire du moyen de contrôle d'accès sont formés d'une seule pièce.

15. Ensemble d'adaptateur suivant l'une quelconque des revendications précédentes, dans lequel ledit premier port proximal de non ventilation de l'adaptateur se trouve dans un alignement essentiellement axial avec le port distal de l'adaptateur.

16. Ensemble d'adaptateur suivant l'une quelconque des revendications précédentes et en combinaison avec un premier cathéter (242) connecté audit premier port d'accès pour aspirer les voies respiratoires lorsqu'on le souhaite, ledit premier cathéter pouvant être positionné d'une façon coulissante à travers le premier port d'accès pour une entrée alignée d'une façon essentiellement axiale à travers l'intérieur de l'adaptateur et dans l'extrémité proximale du tube endotrachéal.

17. Combinaison suivant la revendication 16, dans laquelle l'extrémité distale dudit premier cathéter est connectée d'une façon permanente au premier port d'accès.

18. Combinaison suivant la revendication 16, dans laquelle l'extrémité distale dudit premier cathéter est connectée d'une façon amovible audit premier port d'accès.

19. Combinaison suivant l'une quelconque des revendications 16 à 18, dans laquelle la partie mobile dudit adaptateur comprend un deuxième port d'accès, et la combinaison comprend en outre un deuxième cathéter, connecté audit deuxième port d'accès, en vue de fournir un autre traitement comprenant la délivrance de fluides, l'administration de médicaments, le monitorage, le prélèvement d'échantillons sur le patient ou la réalisation d'une inspection visuelle des voies respiratoires quand on le souhaite, le deuxième cathéter pouvant être positionné d'une façon coulissante à travers le deuxième port d'accès pour une entrée alignée d'une façon essentiellement axiale à travers l'intérieur de l'adaptateur et dans l'extrémité proximale du tube endotrachéal.

20. Combinaison suivant la revendication 19, dans laquelle l'extrémité distale dudit deuxième cathéter est connectée d'une façon permanente audit deuxième port d'accès.

21. Combinaison suivant la revendication 19, dans laquelle l'extrémité distale dudit deuxième cathéter est connectée d'une façon amovible audit deuxième port d'accès.
